(19) **Europäisches Patentamt**

**European Patent Office**

**Office européen des brevets**

(11) **EP 0 532 465 B1**

(12) **EUROPÄISCHE PATENTSCHRIFT**

(45) Veröffentlichungstag und Bekanntmachung des Hinweises auf die Patenterteilung:
**10.07.2002   Patentblatt 2002/28**

(51) Int Cl.⁷: **C07K 14/415**, A61K 38/55, A61K 7/48, A61K 9/70

(21) Anmeldenummer: **92810676.4**

(22) Anmeldetag: **03.09.1992**

(54) **Proteinfraktion zur kosmetischen und dermatologischen Pflege der Haut**

Protein fraction for cosmetic and dermatological care of the skin

Fraction protéinique pour les soins cosmétiques et dermatologiques de la peau

(84) Benannte Vertragsstaaten:
**CH DE ES FR GB IT LI**

(30) Priorität: **13.09.1991  CH 270591**

(43) Veröffentlichungstag der Anmeldung:
**17.03.1993   Patentblatt 1993/11**

(73) Patentinhaber: **Pentapharm A.G.**
**CH-4052 Basel (CH)**

(72) Erfinder:
• **Vögeli, Rainer**
  **CH-4416 Bubendorf (CH)**
• **Stocker, Kurt**
  **CH-4147 Aesch (CH)**

• **Müller, Christian**
  **CH-4163 Reinach (CH)**

(74) Vertreter: **Braun, André, jr.**
**BRAUN & PARTNER**
**Patent-, Marken-, Rechtsanwälte**
**Reussstrasse 22**
**4054 Basel (CH)**

(56) Entgegenhaltungen:
**EP-A- 0 420 600          US-A- 2 794 800**

Bemerkungen:
Die Akte enthält technische Angaben, die nach dem Eingang der Anmeldung eingereicht wurden und die nicht in dieser Patentschrift enthalten sind.

**Beschreibung**

Die Aufgabe der Haut, als ein den Organismus umhüllendes Organ, besteht in abdichtenden und vermittelnden Funktionen gegenüber der Umwelt. Verschiedene biochemische und biophysikalische Systeme dienen der Aufrechterhaltung der Integrität dieses exponierten Organs: Beispielsweise schützt ein Immunsystem die Haut vor Schäden durch pathogene Mikroorganismen, das Melanin bildende System regelt die Pigmentierung und bewahrt die Haut vor Strahlenschäden, ein Lipidsystem produziert Lipidmizellen, die den transdermalen Wasserverlust eindämmen und eine geregelte Keratinsynthese liefert die mechanisch widerstandsfähige Hornschicht. Den genannten Systemen liegen komplexe chemische Prozesse zugrunde, deren Ablauf durch Enzyme in Gang gehalten und durch Enzyminhibitoren geregelt wird. Bereits eine geringfügige Hemmung oder Enthemmung dieser biochemischen Systeme äussert sich in spürbaren Veränderungen der Haut. Der sichtbare und fühlbare Zustand der Haut gilt jedoch als Masstab für Schönheit, Gesundheit und Jugend; ihn zu erhalten ist ein generelles Ziel pflegender Kosmetik.

Der Feuchtigkeitsgehalt der Haut spielt für deren Aussehen, Elastizität und fühlbare Textur eine entscheidende Rolle. Eine dehnbare, elastische Grenzmembran, das Stratum conjunctum, zwischen Hornschicht und lebender Epidermis gelegen, verhindert in der gesunden Haut einen transdermalen Wasserverlust. Diese Permeabilitätsbarriere besteht aus Lipiden, Proteinen und Kohlehydraten und kann dementsprechend durch sehr unterschiedliche Einflüsse geschädigt werden. Beispielsweise kann waschen mit Tensiden Lipide extrahieren und dadurch die Wasserdurchlässigkeit des Stratum conjunctum erhöhen, ultraviolette Strahlung kann eine Quervernetzung oder Spaltung von Proteinen, Proteoglykanen und Polysacchariden katalysieren, dadurch eine Elastizitätsabnahme und eine erhöhte Verletzbarkeit des Stratum conjunctum verursachen, bakterielle Enzyme können einen Abbau von Proteinen und Kohlehydraten des Stratum conjunctum katalysieren und bei entzündlichen Prozessen und Immunreaktionen können im Überschuss mobilisierte körpereigene Enzyme wie zum Beispiel Tryptasen, Elastasen und Cathepsine die Haut und im Besonderen deren Permeabilitätsbarriere angreifen. US-Patent 2,794,800 beschreibt Proteingemische, welche keine Elastase hemmende Aktivität aufweist. EP 0 420 600 beschreibt pharmazeutische Zusammensetzungen, welche zwar gewisse Elastase hemmende Aktivität aufweist. Es wird aber keine Quelle für einen Elastase-Inhibitor angegeben.

Im Bemühen, den Feuchtigkeitsgehalt der Haut zu erhöhen und damit ihre Beschaffenheit zu verbessern, wurden verschiedene kosmetische Hautpflegemittel entwickelt. Eine Übersicht über hydratisierende Kosmetikwirkstoffe findet sich bei S.D. Randazzo und P. Morganti, J. Appl. Cosmetol. 8, 93-102, (1990). Es gelangen hydrophile, hygroskopische Stoffe wie z. B. Glycerin, Sorbit, verschiedene Zucker oder Proteinhydrolysate zur Anwendung. Alle diese Substanzen werden jedoch leicht weggewaschen, etliche hinterlassen eine unangenehmes, klebriges Gefühl auf der Hautoberfläche und andere können, bei besonders ausgeprägten wasseranziehenden Eigenschaften, sogar die Hornschicht zusätzlich austrocknen. Um der Haut vermehrt Feuchtigkeit zuzuführen und zu erhalten, gelangen auch Wasser in Öl-Emulsionen zur Anwendung. Diese hinterlassen aber auf der Hautoberfläche eine dicht schliessende Fettschicht, verursachen einen Wasserstau, eine unangenehme Quellung der Hornschicht und langfristig eine schwer reparierbare Störung der Lipidzusammensetzung des Stratum conjunctum. Hautpflegemittel, welche zur Verstärkung der Permeabilitätsbarriere Glycolipide und Phospholipide aus Rinderhirn erhalten, bewirken einen messbar erhöhten Feuchtigkeitsgehalt in der Haut von Probanden und erzeugen dabei den unerwünschten, undurchlässigen Fettfilm auf der Hornschicht nicht. Da nun aber Lipide aus Rinderhirn potentielle Träger des Erreregers der bovinen spongiformen Encephalopathie (BSE, Rinderwahnsinn) sein könnten, besteht für derartige Produkte, selbst wenn sie nach Verfahren hergestellt werden, die mit Sicherheit den Erreger ausschliessen, nur eine sehr beschränkte Nachfrage.

Um möglicherweise Alternativen zu Wirkstoffen bovinen Ursprungs zu finden, wurde an Probanden die hydratisierende Wirkung verschiedener pflanzlicher Lipide und Proteine untersucht. Es wurde nun gefunden, dass aus Leguminosensamen extrahierte Proteine nach lokaler Applikation den Feuchtigkeitsgehalt der Haut besonders stark und im Vergleich zu bekannten Hydratisierungsmitteln besonders anhaltend erhöhen. Überraschenderweise wurde aber auch gefunden, dass Zubereitungen von Proteinen aus Leguminosensamen bei einigen Probanden den Juckreiz an Insektenstichstellen milderten und bei Probanden die an Psoriasis litten, Juckreiz, Hautrötung und Abschuppung an den Läsionsstellen deutlich verminderten. Bei einer anschliessenden systematischen Untersuchung der entzündungshemmenden Wirkung von Proteinen aus Leguminosensamen wurde ferner gefunden, dass diese Substanzen die durch Trichlorethylen an Probanden erzeugte lokale Hautrötung rascher zum Abklingen bringen als eine analoge Zubereitung ohne Leguminosenproteine und dass die beobachtete entzündungshemmende Wirkung etwa gleich gross war wie diejenige eines Flumethason enthaltenden Kontrollpräparates. Es wurde ferner gefunden, dass die Elastizität von Hautstellen, die mit Proteinen aus Leguminosensamen behandelt wurden, messbar zunahm. Ausserdem wurde gefunden, dass die genannten Proteine aus Leguminosensamen, wie z.B. Tryptase, PMN-Elastase, Fibroblastenelastase und Trypsin, Proteinasen hemmen.

Die vorliegende Erfindung betrifft nun eine Proteinfraktion und diese enthaltende Wirkstoffkonzentrate und Präparate zur Hautpflege und/oder zur Behandlung von entzündlichen Hauterkrankungen, welche mindestens ein aus Leguminosensamen erhältliches Protein oder eine Proteinfraktion mit entzündungshemmender, hydratisierender, die Hautelastizität steigernder, proteinasehemmender Wirkung enthalten.

Als Leguminosensamen zur Gewinnung der erfindungsgemässen Proteine eignen sich Bohnenarten wie zum Beispiel Phaseolus angularis, Phaseolus lunatus, Phaseolus aureus, Phaseolus vulgaris, Phaseolus coccineus, Phaseolus limensis , Erbsenarten wie zum Beispiel Lathyrus odoratus, die Soyabohnen Glycine max und Glycine hisoida, die Erdnuss Arachis hypogaea sowie die Samen tropischer Leguminosen der Gattungen Cajanus, Dolichus, Vigna und Vicia.

Die erfindungsgemässen Proteinfraktionen können aus den genannten Leguminosensamen gewonnen werden, in dem man die getrockneten Samen mahlt, das erhaltene Mehl mit einem organischen Lösungsmittel oder einem Lösungsmittelgemisch extrahiert, trocknet und das derart entfettete Mehl mit Wasser oder einer wässrigen Elektrolytlösung bei einem pH von 2 bis 10, vorzugsweise bei pH 5 bis 6 extrahiert, den Extrakt auf pH 5 bis 7 stellt, im Vacuum einengt, das Konzentrat unter Zusatz eines Filterhilfsmittels wie zum Beispiel Kieselgur klar filtriert oder zentrifugiert, die Proteine daraus entweder durch Salzfällung, beispielsweise mit Ammoniumsulfat bei 30 bis 80% Sättigung, oder durch Fällung mit einem organischen, wassermischbaren Lösungsmittel wie zum Beispiel Ethanol in einer Konzentration von 60 bis 90% abscheidet, durch Filtration oder Zentrifugation sammelt und schliesslich entweder direkt im Vacuum trocknet oder vorerst Salze durch Dialyse, Gelfiltration oder Ultrafiltration entfernt und dann lyophilisiert. Es wird eine Proteinfraktion als Trockensubstanz erhalten, welche in der Polyacrylamidgelelektrophorese in Anwesenheit von Natriumdodecylsulfat (Phastsystem, Pharmacia Biosystems, Uppsala, S) mindestens eine Bande aufweist, deren elektrophoretische Mobilität auf relative Molekularmassen von 3000 bis 30 000 g/mol schliessen lässt. Die erhaltene Proteinfraktion weist einen Gehalt an Gesamtstickstoff von 14 bis 20% und an Aminostickstoff von 1 bis 2% jeweils bezogen auf den Proteingehalt auf, ist in Wasser und wässrigen Elektrolytlösungen löslich, in Ethanol und Aceton unlöslich und zeigt in wässriger Lösung nach Zusatz von Trichloressigsäure, Sulfosalicylsäure, Pikrinsäure oder Benzethoniumchlorid eine starke Ausfällung. Ferner hemmt die Proteinfraktion Proteinasen und zeigt z.B., gemessen an Trypsin, einen $I_{50}$-Wert von unter 10 µg (bezogen auf die Trockensubstanz) pro ml Testgemisch, gemessen an PMN-Elastase, einen $I_{50}$-Wert von unter 100 µg (bezogen auf die Trockensubstanz) pro ml, gemessen an Tryptase, einen $I_{50}$-Wert von unter 200 mg (bezogen auf die Trockensubstanz) pro ml Testgemisch und gemessen an Fibroblastenelastase, einen $I_{50}$-Wert von unter 350 mg (bezogen auf die Trockensubstanz) pro ml Testgemisch. Die erhaltene Proteinfraktion übt in Konzentrationen von 0,1 bis 2% (berechnet als Trockensubstanz) in geeignete kosmetische oder dermatologische Vehikel eingearbeitet, auf der Haut menschlicher Probanden, eine hydratisierende, juckreizstillende, entzündungshemmende und die Hautelastizität steigernde Wirkung aus.

Zur dermatologischen und kosmetischen Anwendung werden die erfindungsgemässen Substanzen zweckmässigerweise in einem hautfreundlichen Vehikel, beispielsweise als Crème, Lotion, Gel, Gesichtsmaske, Puder oder Pflaster eingesetzt. In der Regel enthalten die fertigen Präparate mit Ausnahme der Pflaster 0,01 bis 5 Massen-%, vorzugsweise 0,1 bis 2 Massen-%, des Wirkstoffs als Trockensubstanz. Pflaster zur transdermalen Applikation eines erfindungsgemässen Präparats können hingegen bis zu 90 Massen-% Wirkstoff enthalten. Um die Formulierung flüssiger oder halbfester Zubereitungen zu erleichtern, können aus den Proteinfraktionen gut dosierbare, stabile Wirkstoffkonzentrate mit einem Gehalt an Wirkstoff als Trockensubstanz von 1 bis 15 Massen-%, vorzugsweise etwa 7,5 Massen-%, hergestellt werden, indem man die erfindungsgemässen Proteinfraktionen in Wasser löst und mit einem Zusatz eines wasserlöslichen Konservierungsmittels wie zum Beispiel Methyl-p-oxybenzoat zur Verhütung mikrobiellen Wachstums, eines mehrwertigen Alkohols wie zum Beispiel Ethylenglycol oder Propylenglycol zur Proteinstabilisierung und eines nichtionogenen oder amphoteren Tensids wie zum Beispiel Polysorbat 80, Octoxynol, Cocoamphoglycinat oder Cocoamidopropylbetain zur Unterdrückung hydrophober Wechselwirkung und damit verbundener Proteinausflockung versieht.

Zur Bestimmung der entzündungshemmenden Wirkung der erfindungsgemässen Präparate eignet sich beispielsweise der Trichlorethylen-Erythemtest nach H. Friderich, (Ärztliche Forschung 20, 549-552, 1966) und zu dessen photometrischer Auswertung ein Remmissions-Farbmessgerät wie zum Beispiel das Chroma-Meter CR-300, der Firma Minolta Camera Co., Osaka, Japan.

Die Messung der Hautelastizität kann dadurch erfolgen, dass man die Haut eines Probanden mit einer unter definiertem Unterdruck stehenden Hohlsonde ansaugt, die Eindringtiefe der Haut in die Sonde misst, hierauf den Druck durch Belüftung normalisiert und wiederum die Eindringtiefe der entlasteten Haut in die Sonde misst. Die Geschwindigkeit und der Grad der Rückbildung der durch Unterdruck gedehnten Haut bilden ein Mass für die Hautelastizität. Für die Messung der Hautelastizität nach diesem Prinzip ist das registrierende, mit einem Mikroprozessor ausgestattete Gerät "Cutometer" der Firma Courage & Khazaka electronic GmbH, Köln, D, sehr geeignet. Eine Bestimmung des Hautfeuchtigkeitsgrades kann mit dem elektronischen Messgerät "Corneometer" (Courage & Khazaka) erfolgen. Diesem Messprinzip liegt die Erkenntnis unterschiedlicher Dielektrizitätskonstanten von Wasser und anderen Hautbestandteilen zugrunde.

Zur in vitro Bestimmung der Proteinaseinhibitorwirkung der erfindungsgemässen Präparate wird das zu hemmende Enzym mit dem Inhibitor beziehungsweise mit Placebo während einer definierten Zeitperiode inkubiert, hierauf mit einem chromogenen Proteinasesubstrat versetzt und die durch das verbleibende, nicht gehemmte Enzym pro Zeiteinheit katalysierte p-Nitroanilinfreisetzung photometrisch, bei einer Wellenlänge von 405 nm, als $DA_{405}/t$ gemes-

sen. Aus der Differenz zwischen DA/t Referenz und DA/t Probe lässt sich der $I_{50}$-Wert als die Präparatemenge errechnen, welche unter den definierten Bedingungen das Enzym zu 50% hemmt. Als chromogene Substrate eignen sich für PMN-Elastase und Fibroblastenelastase: MeOSuc-Ala-Ala-Pro-Val-pNA, für Trypsin: Bz-Val-Gly-Arg-pNA und für Tryptase: Tos-Gly-Pro-Arg-pNA (eine ausführliche Beschreibung der Methoden zur Bestimmung von Proteinasen und deren Inhibitoren mittels chromogener Substrate findet sich bei I. Witt, Eur. J. Clin. Chem. Clin. Biol., 29, 355-374, 1991).

Die Proteinaseninhibitorwirkung kann auch im Lysat kultivierter Fibroblasten demonstriert werden. Die Bestimmung erfolgt, wie oben beschrieben, mit einem chromogenen Proteinasensubstrat.

Um die Elastasehemmung in einem ex vivo System aufzuzeigen, kann elastinreiches Gewebe, wie z.B. eine Sehne, in einem Organbad durch Zugabe von PMN-Elastase geschädigt werden. Dies kann durch gleichzeitigen Zusatz des erfindungsgemässen Präparats verhindert werden.

Beispiel 1

Herstellung einer entzündungshemmenden Proteinfraktion aus Sovabohnen

100 g getrocknete Soyabohnen wurden gemahlen, zweimal mit je 300 ml Oktan während jeweils 3 Stunden gerührt, durch eine Nutsche filtriert, gründlich abgepresst und das derart entfettete Material unter Vacuum getrocknet. Das getrocknete Material wurde in 1,35 l entmineralisiertem und 0,5 % des Bakteriostatikums Phenonip$^R$ enthaltendem Wasser suspendiert, der pH-Wert der Suspension wurde mit Salzsäure auf 2,5 bis 3 gestellt, das Gemisch wurde während 2 Std. bei 20°C gerührt und hierauf zentrifugiert. Der opaleszierende Ueberstand wurde auf pH 5,2 gestellt, im Vacuum bei 40 bis 50°C auf 200 ml eingeengt, mit 400 ml gesättigter Ammoniumsulfat-Lösung versetzt und während 15 Std. bei Raumtemperatur stehen gelassen. Die Proteinausfällungen wurden durch Zentrifugation abgetrennt, in 100 ml wässriger Phenoniplösung 0,5% aufgeschlämmt, über Nacht gegen 2 l Phenonipwasser dialysiert, durch Filtration durch eine asbestfreie Filterschicht von ausgeschiedenem Material befreit und schliesslich lyophilisiert. Es wurde eine Proteinfraktion erhalten, welche in der Polyacrylamidgelelektrophorese in Anwesenheit von Natriumdodecylsulfat 4 Banden aufwies, deren elektrophoretische Mobilität auf relative Molekularmassen von 3000 bis 30 000 g/mol schliessen liess. Die erhaltene Proteinfraktion wies einen Gehalt an Gesamtstickstoff von 15,2% und an Aminostickstoff von 1,4% jeweils bezogen auf den Proteingehalt auf, war in Wasser und wässrigen Elektrolytlösungen löslich, in Ethanol und Aceton unlöslich, zeigte in wässriger Lösung nach Zusatz von Trichloressigsäure, Sulfosalicylsäure, Pikrinsäure oder Benzethoniumchlorid eine starke Ausfällung und zeigte, gemessen an Trypsin, einen $I_{50}$-Wert von 3 µg (bezogen auf die Trockensubstanz) pro ml Testgemisch und gemessen an PMN-Elastase, einen $I_{50}$-Wert von 68 µg (bezogen auf die Trokkensubstanz) pro ml. Die erhaltene Proteinfraktion übte in einer Konzentrationen von 0,75% in eine kosmetische Lotion eingearbeitet, auf die Haut menschlicher Probanden appliziert, eine hydratisierende, juckreizstillende, entzündungshemmende und die Hautelastizität steigernde Wirkung aus.

Beispiel 2

Herstellung einer entzündungshemmenden Proteinfraktion aus Limabohnen

100 g getrocknete Limabohnen (Phaseolus lunatus) wurden gemahlen, mit 500 ml Ethanol 95 Vol% während 1 Stunde gerührt, abgenutscht, der feuchte Rückstand zweimal mit je 500 ml Essigsäure 1 N während je 1 Stunde gerührt, und erneut abgenutscht. Die vereinigten Essigsäureextrakte wurden im Vacuum auf 100 ml eingeengt, mit 50 Vol% Ethanol versetzt, ausgefällte Polysaccharide wurden durch Filtration entfernt, aus dem Filtrat wurden die aktiven Proteine durch Erhöhung der Ethanolkonzentration auf 80% ausgefällt, auf einer Nutsche gesammelt und im Vacuum getrocknet. Es wurde eine Proteinfraktion erhalten, welche in der Polyacrylamidgelelektrophorese in Anwesenheit von Natriumdodecylsulfat 5 Banden aufwies, deren elektrophoretische Mobilität auf relative Molekularmassen zwischen 3000 und 30 000 g/mol schliessen liess. Die erhaltene Proteinfraktion wies einen Gehalt an Gesamtstickstoff von 14,9% und an Aminostickstoff von 1,3% jeweils bezogen auf den Proteingehalt auf, war in Wasser und wässrigen Elektrolytlösungen löslich, in Ethanol und Aceton unlöslich, zeigte in wässriger Lösung nach Zusatz von Trichloressigsäure, Sulfosalicylsäure oder Pikrinsäure eine starke Ausfällung und zeigte, gemessen an Trypsin, einen $I_{50}$-Wert von 5µg (bezogen auf die Trockensubstanz) pro ml Testgemisch und gemessen an PMN-Elastase, einen $I_{50}$-Wert von 89 µg (bezogen auf die Trockensubstanz) pro ml. Die erhaltene Proteinfraktion übte in einer Konzentrationen von 1,5% in eine kosmetische Lotion eingearbeitet, auf die Haut menschlicher Probanden appliziert, eine hydratisierende, juckreizstillende, entzündungshemmende und die Hautelastizität steigernde Wirkung aus.

Beispiel 3

Herstellung eines stabilen Wirkstoffkonzentrates

20 g Propylenglycol, 2 g Tween 80 und 0,2 g Methyl-p-oxybenzoat wurden unter Erwärmung auf 70°C in 60 ml dest. Wasser gelöst. Nach dem Abkühlen auf 30°C wurden in dieser Lösung 7,5 g einer nach Beispiel 1 hergestellten Proteinfraktion aufgelöst, der pH-Wert auf 7 gestellt und das Volumen mit dest. Wasser auf 100 ml ergänzt.

Beispiel 4

Bestimmung der entzündungshemmenden Wirkung einer Soyaproteinfraktion

Neun Volumenteile der käuflichen, hautverträglichen wirkstoffreien Emulsion "Excipial U Lotio" (Spirig AG, Egerkingen, CH) wurden mit je einem Volumenteil dest. Wasser (Placebo) bezw. der nach Beispiel 1 hergestellten, in einer Konzentration von 75 mg pro ml in dest. Wasser vorgelösten Probe versetzt und homogenisiert. Bei zwei Probanden wurde die Unterarmhaut an vier Stellen mit je 150 µl auf Patchtest-Pflaster aufgebrachtem Trichlorethylen während 5 Minuten irritiert. Hierauf wurden je zwei der irritierten Hautregionen mit der Placeboemulsion bezw. mit der Probe behandelt. Die Intensität der Hautrötung wurde mit dem Chromameter alle fünf Minuten, während insgesamt 90 Minuten gemessen. Der Verlauf der gemessenen und gemittelten Extinktionswerte ist in Tabelle 1 aufgeführt und zeigt für die Probe im Vergleich zu Placebo, eine wesentlich geringere Rötungsintensität, welche auch rascher wieder zur Norm abklingt.

Tabelle 1

| Zeit (min.) | Extinktion Placebo | Extinktion Probe | Differenz |
|---|---|---|---|
| 5 | 7,20 | 6,90 | 0,30 |
| 10 | 6,98 | 6,82 | 0,16 |
| 15 | 7,04 | 6,93 | 0,11 |
| 20 | 7,06 | 6,47 | 0,59 |
| 25 | 6,55 | 5,71 | 0,84 |
| 30 | 6,03 | 5,39 | 0,64 |
| 35 | 4,91 | 4,56 | 0,35 |
| 40 | 4,58 | 3,99 | 0,56 |
| 45 | 3,65 | 3,10 | 0,55 |
| 50 | 3,82 | 2,47 | 0,81 |
| 55 | 2,85 | 2,22 | 0,63 |
| 60 | 2,38 | 1,83 | 0,55 |
| 65 | 1,96 | 1,40 | 0,56 |
| 70 | 2,15 | 1,29 | 0,86 |
| 75 | 1,74 | 1,00 | 0,74 |
| 80 | 1,25 | 0,93 | 0,32 |
| 90 | 1,29 | 1,29 | 0 |

Beispiel 5

Bestimmung der elastasehemmenden Aktivität einer Limabohnenproteinfraktion

500 µl einer Lösung von MeOSuc-Ala-Ala-Pro-Val-pNA, 1 mM, in albuminhaltigem Puffer pH 7,5, wurde mit je 10 µl von Proben aus einer wässrigen Verdünnungsreihe der nach Beispiel 2 hergestellten Proteinfraktion bezw. mit 10 µl dest. Wasser (Referenz) versetzt und 15 Minuten bei 37°C vorinkubiert. Zu jedem Testansatz wurden je 100 µl PMN-Elastase, 100 nM zugegeben, mit einem registrierenden Photometer die p-Nitronanilin-Freisetzung während 10 Minuten bei 405 nm aufgezeichnet, daraus die Absorptionsdifferenzen pro Minute (DA/min) ermittelt und schliesslich nach der folgenden Gleichung die Elastasehemmung in % errechnet:

$$\% \text{ Hemmung} = \frac{DA_{Ref.}/\min - DA_{Probe}/\min}{DA_{Ref.}/\min} \times 100$$

Aus einer graphischen Dosis-Wirkungskurve wurde ein $I_{50}$-Wert (PMN-Elastase) von 89 mg/l (bezogen auf die Trockensubstanz) für die Proteinfraktion ermittelt.

Beispiel 6

Bestimmung der trypsinhemmenden Aktivität einer entzündungshemmenden Proteinfraktion aus Sovabohnen

0,100 ml Trypsinlösung, 30 E/ml, wurden mit je 0,100 ml von Proben aus einer wässrigen Verdünnungsreihe der nach Beispiel 1 hergestellten Proteinfraktion respektive mit 0,100 ml dest. Wasser (Referenz) versetzt und eine Minute bei Raumtemperatur gehalten. Bei 37°C wurde zu jedem Testansatz 1,7 ml Tris-Imidazolpuffer pH 8,4 und 0,100 ml einer 4 millimolaren Lösung Bz-Val-Gly-Arg-pNA zugesetzt und mit einem registrierenden Photometer die p-Nitroanilin-Freisetzung während 10 Minuten aufgezeichnet, daraus die Absorptionsdifferenzen pro Minute (DA/min) ermittelt und schliesslich nach der folgenden Gleichung die Trypsinhemmung in % errechnet:

$$\% \text{ Hemmung} = \frac{DA_{Ref.}/\min - DA_{Probe}/\min}{DA_{Ref.}/\min} \times 100$$

Aus einer graphischen Dosis-Wirkungskurve wurde ein $I_{50}$-Wert für die Proteinfraktion von 3 mg/l (bezogen auf die Trockensubstanz) ermittelt.

Beispiel 7

Bestimmung der tryptasehemmenden Aktivität einer entzündungshemmenden Proteinfraktion aus Soyabohnen

Zu 1000 µl Tris-HCl Puffer, pH 7,6, unterschiedliche Volumenanteile des nach Beispiel 3 hergestellten, stabilen Wirkstoffkonzentrates enthaltend, wurden 125 µl in Puffer gelöstes Heparin (c = 10 mg/ml) sowie 125 µl Tos-Gly-Pro-Arg-pNA, 1,5 mM pipettiert. Jedem Testansatz wurden 125 µl menschliche Tryptase aus Lunge (c = 28 U/ml) zugesetzt und bei 25 °C mit einem registrierenden Photometer die p-Nitroanilin-Freisetzung während 3 Minuten bei 405 nm aufgezeichnet, daraus die Absorptionsdifferenzen pro Minute (DA/min) ermittelt und schliesslich nach der folgenden Gleichung die Tryptasehemmung in % errechnet:

$$\% \text{ Hemmung} = \frac{DA_{Ref.}/\min - DA_{Probe}/\min}{DA_{Ref.}/\min} \times 100$$

Aus einer graphischen Dosis-Wirkungskurve wurde ein $I_{50}$-Wert für das Wirkstoffkonzentrat von 160 mg/ml ermittelt.

Beispiel 8

Wirkung einer entzündungshemmenden Proteinfraktion in einem ex vivo Testsystem

Eine Sehne, isoliert aus dem Schwanz einer weissen Maus des Stammes NMRI, wurde bei Raumtemperatur in einem Organbad, enthaltend 0,5 ml Tyrodelösung, bei pH 7,4 befestigt. Die Sehne wurde mittels eines Fadens mit einem Hebelarm verbunden, welcher eine Veränderung der Sehnenlänge in ein elektronisches Signal umwandelt, das verstärkt und mit einem Potentiometerschreiber aufgezeichnet wird. Nach zwanzigminütiger Vorinkubation wurde die Tyrodelösung durch ein adäquates Volumen Tyrodelösung eretzt, die einerseits 38 µg PMN-Elastase (=Referenzlösung) und andererseits 38 µg PMN-Elastase sowie 100 µl/ml der nach Beispiel 3 hergestellten Wirkstofflösung (=Testlösung) enthielt. Im Falle der Referenzlösung wurde eine kontinuierliche Verlängerung der Sehne registriert, welche in der Folge nach 132 Minuten riss. Bei der Testlösung wurde hingegen keine Veränderung der Sehnenlänge beobachtet.

Beispiel 9

Bestimmung der elastasehemmenden Wirkung einer entzündungshemmenden Proteinfraktion in Fibroblastenkulturen

Swiss 3T3 Mausfibroplasten mit einer Zelldichte von $10^8$ Zellen pro 175 cm$^2$ wurden mit 10 ml 50 mM Tris-HCl bei pH 8,0 enthaltend 1 % Triton X-100 während 30 Minuten bei 4 °C lysiert. Die Zelltrümmer wurden mittels Zentrifugation während 15 Minuten bei 5000 g und 0 °C abgetrennt. 500 µl Lysat wurden mit 500 µl unterschiedlicher Konzentrationen des Wirkstoffkonzentrats, hergestellt nach Beispiel 3, und 34 µl einer Lösung von MeOSuc-Ala-Ala-Pro-Val-pNA versetzt und die p-Nitroanilin-Freisetzung mit einem registrierenden Photometer während 2 Stunden bei 405 nm aufgezeichnet, daraus die Extinktionsdifferenzen pro Minute (DA/min) ermittelt und schliesslich nach der folgenden Gleichung die Elastasehemmung in % errechnet:

$$\% \text{ Hemmung} = \frac{DA_{Ref.}/\min - DA_{Probe}/\min}{DA_{Ref.}/\min} \times 100$$

Aus einer graphischen Dosis-Wirkungskurve wurde ein $I_{50}$-Wert (Fibroblasten-Elastase) von 280 mg/ml für das Wirkstoffkonzentrat ermittelt.

Beispiel 10

Herstellung einer kosmetischen Crème (Oel-in-Wasser-Emulsion)

| | |
|---|---|
| Wirkstoffkonzentrat nach Beispiel 3 | 10,0 g |
| Polysorbat 60 | 3,0 g |
| Sorbitan stearat | 2,0 g |
| Cetylalkohol | 3,0 g |
| Stearinsäure | 6,0 g |
| Isopropylmyristat | 10,0 g |
| Capryl-Caprinsäure-Triglyceride | 5,0 g |
| Phenonip$^R$ | 0,5 g |
| entmineralisiertes Wasser | 56,2 g |
| Propylenglycol | 4,0 g |
| Imidazolinidylharnstoff | 0,3 g |

Alle Komponenten ausser dem Wirkstoffkonzentrat werden bei 70°C unter starkem Rühren vermischt, erkalten gelassen und hierauf mit dem Wirkstoffkonzentrat vermengt.

Beispiel 11

Herstellung einer Lotion zur Behandlung entzündlicher Hautkrankheiten

| | |
|---|---|
| Wirkstoffkonzentrat gemäss Beispiel 3 | 5,0 g |
| Stearylalkohol | 1,0 g |
| Cetearat 6 | 1,0 g |
| Cetearylalkohol | 7,0 g |
| Mineralöl | 8,0 g |
| Cetylalkohol | 1,0 g |
| Glycerylstearat | 2,5 g |
| Phenonip$^R$ | 0,3 g |
| entmineralisiertes Wasser | 72,0 g |

(fortgesetzt)

| | |
|---|---|
| Propylenglycol | 2,0 g |
| Imidazolidinylharnstoff | 0,2 g |

Alle Komponenten ausser dem Wirkstoffkonzentrat werden bei 70°C unter starkem Rühren vermischt, erkalten gelassen und hierauf mit dem Wirkstoffkonzentrat vermengt.

Beispiel 12

Herstellung eines Hautpflege-Gels

| | |
|---|---|
| Wirkstoffkonzentrat nach Beispiel 3 | 5,0 g |
| entmineralisiertes Wasser | 88,5 g |
| Phenonip[R] | 0,3 g |
| Imidazolinylharnstoff | 0,2 g |
| Propylenglycol | 5,0 g |
| Cellulosegummi | 1,0 g |

Alle Komponenten werden im entmineralisierten Wasser bei 40°C unter Rühren gelöst.

Beispiel 13

Wirkung einer Soyaproteinfraktion auf den Feuchtigkeitsgehalt der Haut

An 5 Probanden wurde an fünf Tagen, während einer Testperiode von sieben Tagen, je 60 µl Wirkstoffkonzentrat nach Beispiel 3 auf 20 cm$^2$ Haut auf der Innenseite des linken Unterarms verteilt; der unbehandelte rechte Arm diente jeweils als Kontrolle. Zwei Stunden nach der Applikation wurde die Hautfeuchtigkeit, ausgedrückt in Corneo-Units (CU) gemessen und aus den Messdaten die Steigerung des Hautfeuchtigkeitsgehaltes in Prozent gegenüber dem Ausgangswert berechnet. Die Resultate sind in Tabelle 2 als Durchschnittswerte von fünf Probanden dargestellt.

Tabelle 2

| Tag | Corneo Units | | Steigerung (%) | |
|---|---|---|---|---|
| | Probe | Kontrolle | Probe | Kontrolle |
| 1 | 61,4 | 59,33 | 0 | 0 |
| 3 | 82,4 | 63,6 | 34,2 | 7,2 |
| 4 | 88,8 | 67 | 44,6 | 12,9 |
| 5 | 89,2 | 64,8 | 45,3 | 9,2 |
| 6 | 88 | 60,2 | 43,3 | 1,5 |
| 7 | 85 | 62,4 | 38,4 | 5,2 |

Beispiel 14

Wirkung einer Soyabohnenfraktion auf die Elastizität der Haut

5 Probanden wurde zweimal täglich 60 µl des Wirkstoffkonzentrat nach Beispiel 3 am linken Augenwinkel appliziert; der unbehandelte rechte Augenwinkel diente jeweils als Kontrolle. Vor Beginn sowie 14 Tage nach der Behandlung wurde die Elastizität der Haut ermittelt, indem die Eindringtiefe der Haut in eine unter Unterdruck stehende Hohlsonde sowie die Rückbildung der Haut unter Normaldruck gemessen wurde. Die Differenz der Eindringtiefe von fünfmal nacheinander belasteter und wieder entlasteter Haut (=ds) ist ein Mass für die Hautelastizität. Je kleiner die Differenz ist, desto grösser ist die Elastizität. Die Resultate sind in Tabelle 3 als Durchschnittswerte von fünf Probanden dargestellt.

Tabelle 3

|  | ds links (Probe) | ds rechts (Kontrolle) |
|---|---|---|
| vor Behandlung | 0,067 mm | 0,057 mm |
| nach Behandlung | 0,042 mm | 0,058 mm |

**Patentansprüche**

1. Proteinfraktion enthaltend mindestens ein Protein, **gekennzeichnet durch**

   a) seine Isolierung aus Leguminosensamen,
   b) mindestens eine Bande in der Polyacrylamidgelelektrophorese mit Natriumdodecylsulfat,
   c) Molekularmassen von 3000 bis 30'000 g/mol,
   d) Gehalt an Gesamtstickstoff von 14% bis 20% und Aminostickstoff von 1% bis 2% bezogen auf den Proteingehalt,
   e) Löslichkeit in Wasser und wässrigen Elektrolytlösungen und Unlöslichkeit in Ethanol und Aceton,
   f) starke Ausfällung in wässriger Lösung nach Zusatz von Trichloressigsäure, Sulfosalicylsäure, Pikrinsäure oder Benzethoniumchlorid,
   g) Hemmung von PMN-Elastase und Fibroblastenelastase.

2. Proteinfraktion nach Anspruch 1, **dadurch gekennzeichnet, dass** die Leguminosensamen Soya- oder Limabohnen sind.

3. Verfahren zur Isolierung der Proteinfraktion nach Anspruch 1 oder 2, **gekennzeichnet durch** die folgenden Schritte:

   a) Mahlen der getrockneten Samen,
   b) Entfetten des Mehls **durch** Extrahieren mit einem organischen Lösungsmittel oder Lösungsmittelgemisch,
   c) Extrahieren des Mehls mit Wasser oder einer wässrigen Elektrolytlösung bei pH 5 bis 6,
   d) Einengen des Extrakts bei pH 5 bis 7 und Filtrieren oder Zentrifugieren des Konzentrats,
   e) Abscheiden der darin befindlichen Proteine **durch** Fällung,
   f) Filtrieren oder Zentrifugieren und anschliessendes Trocknen oder Lyophilisieren.

4. Stabiles wässriges Wirkstoffkonzentrat, enthaltend 1 bis 15 Massen-%, bezogen auf die Masse des Wirkstoffkonzentrats, vorzugsweise 7,5 Massen-%, einer Proteinfraktion nach einem der Ansprüche 1 oder 2 und mindestens ein wasserlösliches Konservierungsmittel und/oder mindestens einen mehrwertigen Alkohol und/oder ein nichtionogenes oder amphoteres Tensid.

5. Wirkstoffkonzentrat nach Anspruch 4, enthaltend als wasserlösliches Konservierungsmittel Methyl-p-oxybenzoat, als mehrwertigen Alkohol Propylenglycol und als Tensid Polysorbat 80 und/oder Octoxynol und/oder Cocoamphoglycinat.

6. Verwendung einer Proteinfraktion nach einem der Ansprüche 1 oder 2 oder eines Wirkstoffkonzentrats nach einem der Ansprüche 4 oder 5 zur Herstellung von Präparaten zur Hautpflege und/oder zur Behandlung entzündlicher Hauterkankungen.

7. Präparate zur Hautpflege und/oder Behandlung entzündlicher Hauterkrankungen, enthaltend eine Proteinfraktion nach einem der Ansprüche 1 oder 2 in einer Menge von 0.01 bis 5 Massen-%, vorzugsweise von 0.1 bis 2 Massen-% Trockensubstanz, bezogen auf die Gesamtmasse des Präparats.

8. Auf einem Pflaster zur transdermalen Applikation aufgebrachtes Präparat, das eine Proteinfraktion nach einem der Ansprüche 1 oder 2 in einer Menge von bis zu 90 Massen-% enthält.

**Claims**

1. Protein fraction which contains at least one protein which comprises

a) its isolation from Leguminosae seeds,
b) at least one band in polyacrylamide gel electrophoresis with sodium dodecyl sulfate,
c) molecular weights from 3,000 to 30,000 g/mol,
d) a content of total nitrogen of 14% to 20% and amino nitrogen of 1% to 2% related to the protein content,
e) solubility in water and aqueous electrolyte solutions and insolubility in ethanol and acetone,
f) strong precipitation in aqueous solution after addition of trichloracetic acid, sulfosalicylic acid, picric acid or benzethonium chloride,
g) inhibition of PMN elastase and fibroblast elastase.

2. Protein fraction according to claim 1 wherein the Leguminosae seeds are soya or lima beans.

3. Method for isolating the protein fraction according to claim 1 or 2 which comprises the following steps:

a) grinding of the dried seeds,
b) degreasing of the powder by extraction with an organic solvent or mixture of solvents,
c) extraction of the powder with water or an aqueous electrolyte solution at pH 5 to 6,
d) concentration of the extract at pH 5 to 7 and filtration or centrifugation of the concentrate,
e) separation of the present proteins by precipitation,
f) filtration or centrifugation and subsequent drying or lyophilization.

4. Stable aqueous concentrate of active substances which contains 1 to 15 weight %, related to the weight of the active concentrate, preferably 7.5 weight %, of a protein fraction according to one of claims 1 or 2 and at least one water-soluble preservative and/or at least a polyvalent alcohol and/or a non-ionogenic or amphoteric tenside.

5. Concentrate of active substances according to claim 4 which contains methyl-p-oxybenzoate as the water-soluble preservative, propylene glycol as the polyvalent alcohol and polysorbate 80 and/or octoxynol and/or cocoamphoglycinate as the tenside.

6. Use of a protein fraction according to one of claims 1 or 2 or of a concentrate of active substances according to one of claims 4 or 5 for the manufacture of preparations for skin care and/or treatment of inflammatory skin diseases.

7. Preparations for skin care and/or treatment of inflammatory skin diseases which comprise a protein fraction according to one of claims 1 or 2 in a quantity of 0.01 to 5 weight %, preferably 0.1 to 2 weight % dry substance, based on the total weight of the preparation.

8. The preparation applied on a plaster for transdermal application wherein the preparation contains a protein fraction according to one of claims 1 or 2 in a quantity of up to 90 weight %.

**Revendications**

1. Fraction protéinique contenant au moins une protéine, **caractérisée par**

a) son isolation à partir de graines de légumineuses,
b) au moins une bande apparaissant par électrophorèse sur gel de polyacrylamide avec du dodécyl sulfate de sodium,
c) des poids moléculaires de 3.000 à 30.000 g/mole,
d) une teneur en azote total de 14% à 20% et en amino azote de 1% à 2% par rapport à la teneur en protéine,
e) sa solubilité dans l'eau et les solutions d'électrolytes aqueuses et son insolubilité dans l'éthanol et l'acétone,
f) une forte précipitation dans une solution aqueuse après addition d'acide trichloracétique, d'acide sulfosalicylique, d'acide picrique ou de chlorure de benzéthonium,
g) une inhibition d'élastase de PMN et d'élastase de fibroblastes.

2. Fraction protéinique selon la revendication 1, **caractérisée par le fait que** les graines de légumineuses sont des graines de soja ou de Lima.

3. Méthode d'isolation de la fraction protéinique selon la revendication 1 ou 2, **caractérisée par** les étapes suivantes:

    a) broyage des graines sèches,
    b) dégraissage de la farine par extraction avec un solvant ou mélange de solvants organique,
    c) extraction de la farine avec de l'eau ou une solution d'électrolytes aqueuse entre pH 5 et 6,
    d) concentration de l'extrait entre pH 5 et 7 et filtration ou centrifugation du concentré,
    e) séparation des protéines présentes par précipitation,
    f) filtration ou centrifugation, puis séchage ou lyophilisation.

4. Concentré de principes actifs aqueux et stable contenant de 1 à 15% en masse, par rapport à la masse du concentré de principes actifs, de préférence 7,5% en masse, d'une fraction protéinique selon l'une des revendications 1 ou 2 et au moins un conservateur hydrosoluble et/ou au moins un alcool polyvalent et/ou un tensio-actif non-ionogène ou amphotère.

5. Concentré de principes actifs selon la revendication 4 contenant du méthyl-p-oxybenzoate comme conservateur hydrosoluble, du propylène glycol comme alcool polyvalent et du polysorbate 80 et/ou du octoxynol et/ou du cocoamphoglycinate comme tensio-actif.

6. Utilisation d'une fraction protéinique selon l'une des revendications 1 ou 2 ou d'un concentré de principes actifs selon l'une des revendications 4 ou 5 pour la préparation de produits de soins pour la peau et/ou de traitement de maladies cutanées inflammatoires.

7. Préparations de soins pour la peau et/ou de traitement de maladies cutanées inflammatoires, contenant une fraction protéinique selon l'une des revendications 1 ou 2 dans une quantité de 0,01 à 5% en masse, de préférence de 0,1 à 2% en masse de substance sèche, par rapport à la masse totale de la préparation.

8. Préparation répartie sur un emplâtre d'application transdermique, contenant une fraction protéinique selon l'une des revendications 1 ou 2 dans une quantité pouvant atteindre 90% en masse.